# EUROPEAN PATENT SPECIFICATION

(11) **EP 3 636 780 B1**
(45) Date of publication and mention of the grant of the patent: **15.11.2023**
(21) Application number: 18805473.8
(22) Date of filing: 25.04.2018
(51) Int. Cl.: C12Q 1/6883, C12Q 1/689

(54) **METHOD FOR DIAGNOSING AUTISM BY ANALYZING BACTERIAL METAGENOME**
VERFAHREN ZUR DIAGNOSE VON AUTISMUS DURCH ANALYSE VON BAKTERIELLEM METAGENOM
PROCÉDÉ DE DIAGNOSTIC DE L'AUTISME PAR ANALYSE DU MÉTAGÉNOME BACTÉRIEN

(30) Priority: 26.05.2017 KR 20170065329; 24.04.2018 KR 20180047608
(43) Date of publication of application: 15.04.2020
(73) Proprietor: MD Healthcare Inc., Seoul 03923 (KR)
(72) Inventor: KIM, Yoon-Keun, Paju-si Gyeonggi-do 10908 (KR)
(74) Representative: Dehns
(86) International application number: PCT/KR2018/004795
(87) International publication number: WO 2018/216912

(56) References cited:
- WO-A1-2010/147714
- KR-A- 20110 025 603
- KR-A- 20160 073 157
- US-A1- 2014 065 132
- MARIA DE ANGELIS ET AL: "Autism spectrum disorders and intestinal microbiota", GUT MICROBES, vol. 6, no. 3, 2 April 2015 (2015-04-02), pages 207-213, XP055265045, United States ISSN: 1949-0976, DOI: 10.1080/19490976.2015.1035855
- JAE YOUNG YOO ET AL: "16S rRNA gene-based metagenomic analysis reveals differences in bacteria-derived extracellular vesicles in the urine of pregnant and non-pregnant women", EXPERIMENTAL & MOLECULAR MEDICINE, vol. 48, no. 2, 5 February 2016 (2016-02-05), page e208, XP055321074, DOI: 10.1038/emm.2015.110
- SON, JOSHUA S. et al.: "Comparison of Fecal Microbiota in Children with Autism Spectrum Disorders and Neurotypical Siblings in the Simons Simplex Collection", PLOS One, vol. 10, no. 10, October 2015 (2015-10), pages 1-19, XP055562667,
- LEE, YIINJIN: "Rapid Assessment of Microbiota Changes in Individuals with Autism Spectrum Disorder Using Bacteria-derived Membrane Vesicles in Urine", Experimental Neurobiology, vol. 26, no. 5, October 2017 (2017-10), pages 307-317, XP055562669,

## Description

### [Technical Field]

The present invention relates to a method for diagnosing autism through a bacterial metagenomic analysis and, more specifically, to a method of diagnosing autism by performing a bacterial metagenomic analysis using normal individual-derived and subject-derived urine samples to analyze an increase or decrease in the content of specific bacteria-derived extracellular vesicles.

### [Background Art]

Autism refers to a neurodevelopmental disorder that reduces the ability to understand communication and social interactions. The cause of this symptom has not been clearly elucidated yet, but several researchers speculate that the cause is not an emotional cause, but a genetic developmental disorder. The probability of onset is one in every 1,000 people, and the ratio of men to women is approximately 4 : 1 according to US standards. A 2011 survey shows that the prevalence rate of autism in Korea is 2% or more, and thus has received attention. Autism is generally referred to as 'autism spectrum disorder', and this disorder has been discussed in three separate sections in the academic world. The first is autism (Kanner's Syndrome) referring to children as defined by Leo Kanner, the second is Asperger's Syndrome discussed by Hans Asperger, and the last is high functional autism including Savant Syndrome.

Growing babies generally show social activities such as staring at people, paying attention to voices, grasping fingers, or laughing or smiling. However, autistic babies do not like face to face interaction, and suffer from great difficulties in understanding human societal interactions. In relation to communication, ordinary babies say words on their first birthday, react when their names are called, show actions by indicating toys with their fingers when the babies want the toys, whereas autistic babies choose a different path in the development of communication. Even though some people with autism have a sufficient ability to read and write, people with autism remain silent throughout their lives, and instead, they prefer other methods, that is, methods such as painting or sign language.

In relation to the cause of autism, some theories asserted that autism seemed to be acquired at an early stage of life, but currently, it is predominantly thought that autism is caused by a congenital cause. Usually, abnormalities in brain structure, genetic defects, and abnormalities in neurotransmitters have been identified as the cause of autism. Further, fetal alcohol syndrome, in which a kid is adversely affected by a pregnant woman who likes drinking, was also identified as a cause of autism. However, there is still an ongoing debate about the exact causal factors.

A microbiota or microbiome is a microbial community that includes bacteria, archaea, and eukaryotes present in a given habitat. The intestinal microbiota is known to play a vital role in human's physiological phenomena and significantly affect human health and diseases through interactions with human cells. Bacteria coexisting in human bodies secrete nanometer-sized vesicles to exchange information about genes, proteins, and the like with other cells. The mucous membranes form a physical barrier membrane that does not allow particles with the size of 200 nm or more to pass therethrough, and thus bacteria symbiotically living in the mucous membranes are unable to pass therethrough, but bacteria-derived extracellular vesicles have a size of approximately 100 nm or less and thus relatively freely pass through the mucous membranes and are absorbed into the human body.

Metagenomics, also called environmental genomics, may be analytics for metagenomic data obtained from samples collected from the environment (Korean Patent Publication No. 2011-073049). Recently, the bacterial composition of human microbiota has been listed using a method based on 16s ribosomal RNA (16s rRNA) base sequences, and 16s rDNA base sequences, which are genes of 16s ribosomal RNA, are analyzed using a next generation sequencing (NGS) platform. However, in the onset of autism, identification of causative factors of autism through metagenomic analysis of bacteria-derived vesicles isolated from a human-derived substance, such as urine and the like, and a method of predicting autism have never been reported.

Maria De Angelis et al., (2015) Gut Microbes 6(3):207-213 discloses altered fecal microbiota and metabolomes in autistic subjects compared with healthy controls.

WO2010/147714 discloses a method of diagnosing autism comprising detecting an alteration in the expression of a carbohydrate metabolic enzyme protein, or carbohydrate transporter protein in a subject as compared to a non-autistic subject, and optionally further detecting an altered level of gut bacteria.

Jae Young Yoo et al., (2016) Experimental & Molecular Medicine 48(2): e208 discloses that there are differences in bacteria-derived extracellular vesicles in the urine of pregnant and non-pregnant women.

### [Technical Problem]

The present inventors extracted genes from bacteria-derived extracellular vesicles present in urine as normal individual-derived and subject-derived samples and performed a metagenomic analysis in this regard in order to diagnose the causal factors and risk of autism in advance, and as a result, identified bacteria-derived extracellular vesicles which may act as a causal factor of autism, thereby completing the present invention based on this.

Therefore, an object of the present invention is to provide a method of providing information for diagnosing autism and a method of diagnosing autism, through the metagenomic analysis of bacteria-derived extracellular vesicles.

### [Technical Solution]

The present invention provides a method for providing information for diagnosing autism, and a method for diagnosing autism as defined in the appended set of claims. To achieve the above-described object of the present invention, there is provided a method of providing information for autism diagnosis, comprising the following processes:
(a) extracting DNAs from extracellular vesicles isolated from normal individual and subject urine samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR,

wherein process (c) comprises comparing an increase or decrease in content of extracellular vesicles derived from
one or more bacteria selected from the group consisting of the phylum *Armatimonadetes* and the phylum *Thermi;*
one or more bacteria selected from the group consisting of the class *Fimbriimonadia,* the class *Alphaproteobacteria,* and the class *Deinococci;*
one or more bacteria selected from the group consisting of the order *Fimbriimonadales,* the order *Rhizobiales,* the order *Sphingomonadales,* and the order *Desulfovibrionales;*
one or more bacteria selected from the group consisting of the family *Fimbriimonadaceae,* the family *Rhizobiaceae,* the family *Alcaligenaceae,* the family *Sphingomonadaceae,* and the family *Desulfovibrionaceae; or*
one or more bacteria selected from the group consisting of the genus *Fimbriimonas,* the genus *Achromobacter,* the genus *Roseateles,* the genus *Agrobacterium,* the genus *Sphingomonas,* the genus *Desulfovibrio*, the genus *Halomonas,* and the genus *Jeotgalicoccus*
wherein in process (c), in comparison with the normal individual-derived sample, an increase in the content of the following is diagnosed as autism:
   extracellular vesicles derived from bacteria of the phylum *Thermi,*
   extracellular vesicles derived from bacteria of the class *Deinococci,*
   extracellular vesicles derived from bacteria of the order *Desulfovibrionales,*
   extracellular vesicles derived from bacteria of the family *Desulfovibrionaceae,* or
   extracellular vesicles derived from one or more bacteria selected from the group consisting of the genus *Desulfovibrio*, the genus *Halomonas,* and the genus *Jeotgalicoccus;* or
   wherein in process (c), in comparison with the normal individual-derived sample, a decrease in the content of the following is diagnosed as autism:
      extracellular vesicles derived from bacteria of the phylum *Armatimonadetes,*
      extracellular vesicles derived from one or more bacteria selected from the group consisting of the class *Fimbriimonadia* and the class *Alphaproteobacteria,*
      extracellular vesicles derived from one or more bacteria selected from the group consisting of the order *Fimbriimonadales,* the order *Rhizobiales,* and the order *Sphingomonadales,*
      extracellular vesicles derived from one or more bacteria selected from the group consisting of the family *Fimbriimonadaceae,* the family *Rhizobiaceae,* the family *Alcaligenaceae*, and the family *Sphingomonadaceae,* or
      extracellular vesicles derived from one or more bacteria selected from the group consisting of the genus *Fimbriimonas,* the genus *Achromobacter,* the genus *Roseateles,* the genus *Agrobacterium,* and the genus *Sphingomonas.*

The present invention also provides a method of diagnosing autism, comprising the following processes:
(a) extracting DNAs from extracellular vesicles isolated from normal individual and subject urine samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR,

wherein process (c) comprises comparing an increase or decrease in content of extracellular vesicles derived from
one or more bacteria selected from the group consisting of the phylum *Armatimonadetes* and the phylum *Thermi;*
one or more bacteria selected from the group consisting of the class *Fimbriimonadia,* the class *Alphaproteobacteria,* and the class *Deinococci;*
one or more bacteria selected from the group consisting of the order *Fimbriimonadales*, the order *Rhizobiales,* the order *Sphingomonadales,* and the order *Desulfovibrionales;*
one or more bacteria selected from the group consisting of the family *Fimbriimonadaceae,* the family *Rhizobiaceae,* the family *Alcaligenaceae*, the family *Sphingomonadaceae,* and the family *Desulfovibrionaceae; or*
one or more bacteria selected from the group consisting of the genus *Fimbriimonas,* the genus *Achromobacter,* the genus *Roseateles,* the genus *Agrobacterium,* the genus *Sphingomonas,* the genus *Desulfovibrio*, the genus *Halomonas,* and the genus *Jeotgalicoccus;*
wherein in process (c), in comparison with the normal individual-derived sample, an increase in the content of the following is diagnosed as autism:
   extracellular vesicles derived from bacteria of the phylum *Thermi,*
   extracellular vesicles derived from bacteria of the class *Deinococci,*
   extracellular vesicles derived from bacteria of the order *Desulfovibrionales,*
   extracellular vesicles derived from bacteria of the family *Desulfovibrionaceae,* or
   extracellular vesicles derived from one or more bacteria selected from the group consisting of the genus *Desulfovibrio*, the genus *Halomonas,* and the genus *Jeotgalicoccus;* or
   wherein in process (c), in comparison with the normal individual-derived sample, a decrease in the content of the following is diagnosed as autism:
      extracellular vesicles derived from bacteria of the phylum *Armatimonadetes,*
      extracellular vesicles derived from one or more bacteria selected from the group consisting of the class *Fimbriimonadia* and the class *Alphaproteobacteria,*
      extracellular vesicles derived from one or more bacteria selected from the group consisting of the order *Fimbriimonadales,* the order *Rhizobiales,* and the order *Sphingomonadales,*
      extracellular vesicles derived from one or more bacteria selected from the group consisting of the family *Fimbriimonadaceae,* the family *Rhizobiaceae,* the family *Alcaligenaceae*, and the family *Sphingomonadaceae,* or
      extracellular vesicles derived from one or more bacteria selected from the group consisting of the genus *Fimbriimonas,* the genus *Achromobacter,* the genus *Roseateles,* the genus *Agrobacterium,* and the genus *Sphingomonas.*

In another embodiment of the present invention, process (c) may comprise comparing an increase or decrease in content of extracellular vesicles derived from one or more bacteria selected from the group consisting of the phylum *Armatimonadetes* and the phylum *Thermi.*

In another embodiment of the present invention, process (c) may comprise comparing an increase or decrease in content of extracellular vesicles derived from one or more bacteria selected from the group consisting of the class *Fimbriimonadia,* the class *Alphaproteobacteria,* and the class *Deinococci.*

In another embodiment of the present invention, process (c) may comprise comparing an increase or decrease in content of extracellular vesicles derived from one or more bacteria selected from the group consisting of the order *Fimbriimonadales,* the order *Rhizobiales,* and the order *Sphingomonadales,* and the order *Desulfovibrionales.*

In another embodiment of the present invention, process (c) may comprise comparing an increase or decrease in content of extracellular vesicles derived from one or more bacteria selected from the group consisting of the family *Fimbriimonadaceae,* the family *Rhizobiaceae,* the family *Alcaligenaceae*, the family *Sphingomonadaceae,* and the family *Desulfovibrionaceae.*

In another embodiment of the present invention, process (c) may comprise comparing an increase or decrease in content of extracellular vesicles derived from one or more bacteria selected from the group consisting of the genus *Fimbriimonas,* the genus *Achromobacter,* the genus *Roseateles,* the genus *Agrobacterium,* the genus *Sphingomonas,* the genus *Desulfovibrio*, the genus *Halomonas,* and the genus *Jeotgalicoccus.*

### [Advantageous Effects]

The extracellular vesicles secreted from microorganisms such as bacteria and archaebacteria present in the environment are absorbed into the human body and thus may directly affect the occurrence of inflammation, and before symptoms appear, it is difficult to early diagnose autism characterized by inflammation, and thus it is difficult to efficiently treat autism. Therefore, by diagnosing autism onset in advance through the metagenomic analysis of bacteria-derived extracellular vesicles using human body-derived urine samples according to the present invention, it is possible to diagnose autism early, and to delay the onset time or prevent the onset of autism through proper management. In addition, even after the onset, since it is possible to diagnose autism early, there are advantages in that it is possible to lower the incidence of autism and increase therapeutic effects, and it is possible to ameliorate the progression of the disease or prevent recurrence by avoiding exposure to causal factors through a metagenomic analysis in a patient diagnosed with autism.

### [Description of Drawings]

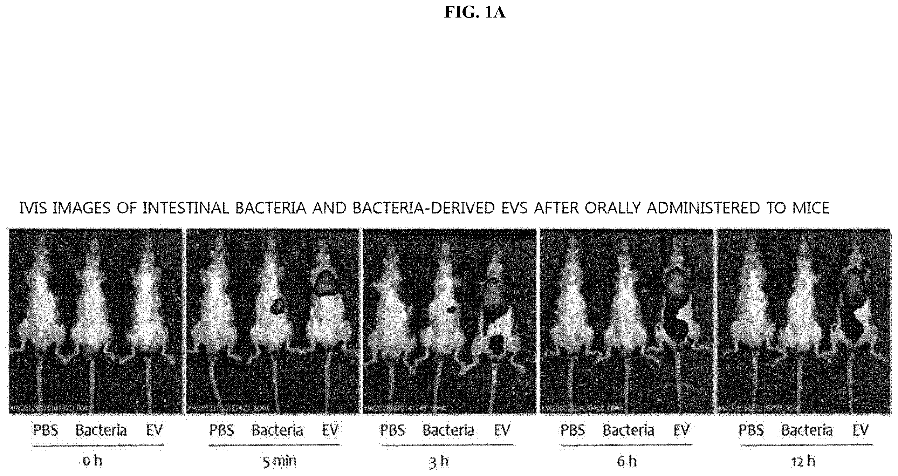

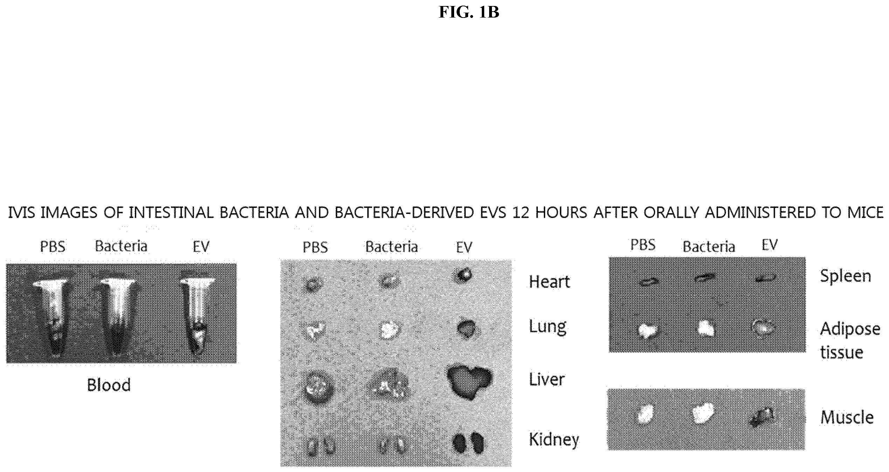

FIG. 1A illustrates images showing the distribution pattern of bacteria and extracellular vesicles over time after intestinal bacteria and bacteria-derived extracellular vesicles (EVs) were orally administered to mice, and FIG. 1B illustrates images showing the distribution pattern of bacteria and EVs after being orally administered to mice and, at 12 hours, blood and various organs were extracted.

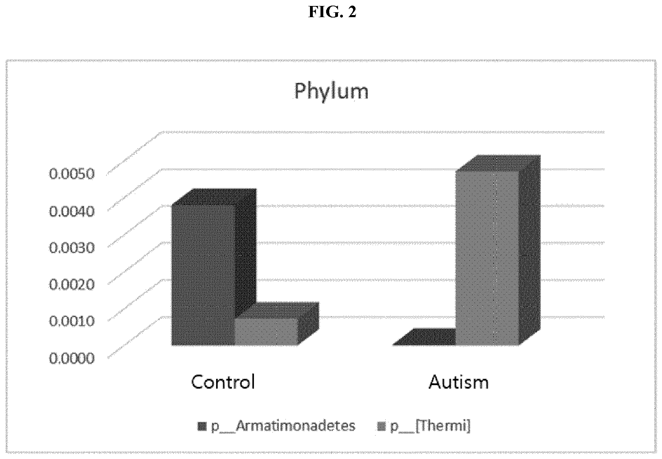

FIG. 2 is a result showing the distribution of bacteria-derived extracellular vesicles (EVs), which is significant in diagnostic performance at the phylum level by isolating bacteria-derived vesicles from urine of a patient with autism and a normal individual, and then performing a metagenomic analysis.

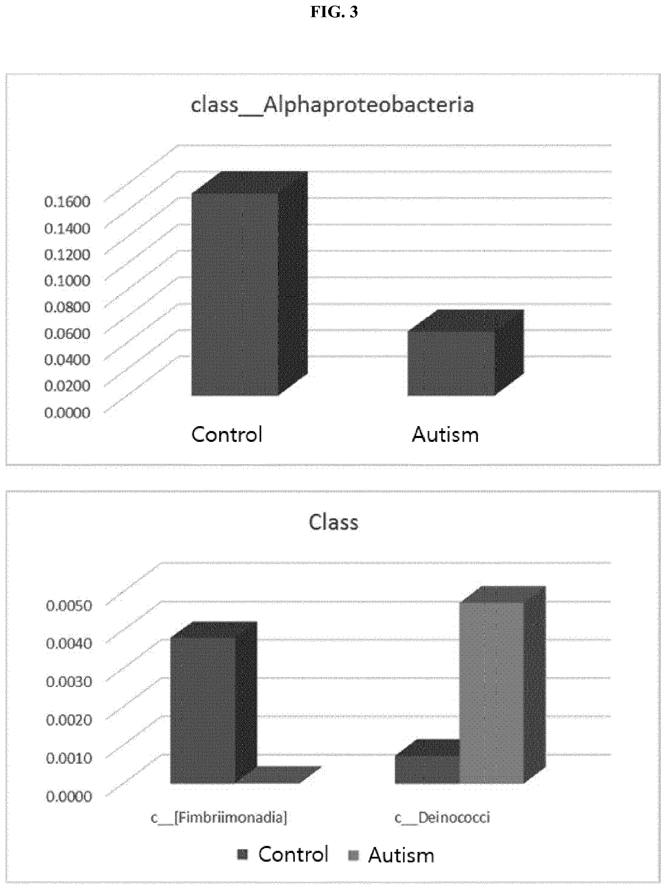

FIG. 3 is a result showing the distribution of bacteria-derived extracellular vesicles (EVs), which is significant in diagnostic performance at the class level by isolating bacteria-derived vesicles from urine of a patient with autism and a normal individual, and then performing a metagenomic analysis.

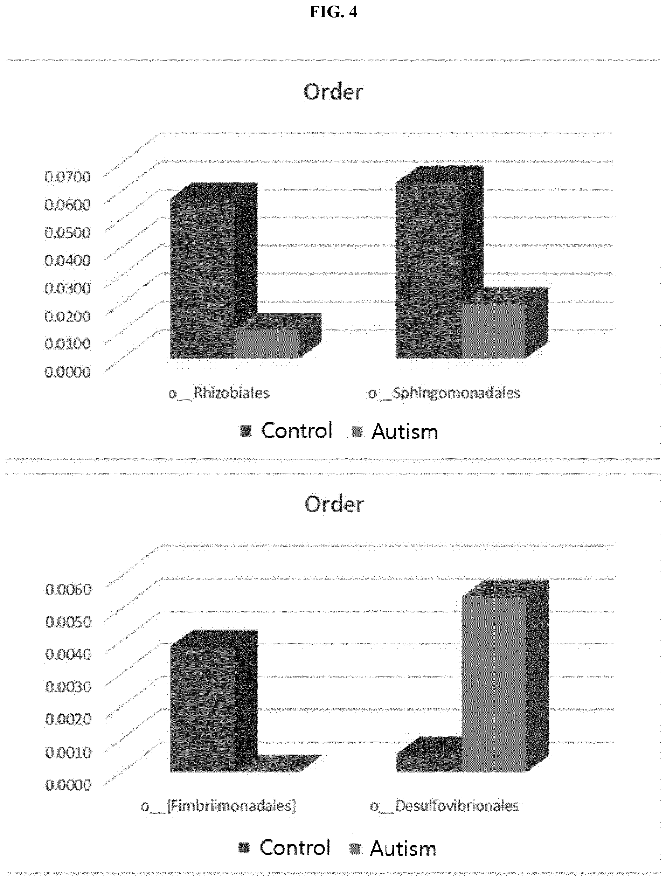

FIG. 4 is a result showing the distribution of bacteria-derived extracellular vesicles (EVs), which is significant in diagnostic performance at the order level by isolating bacteria-derived vesicles from urine of a patient with autism and a normal individual, and then performing a metagenomic analysis.

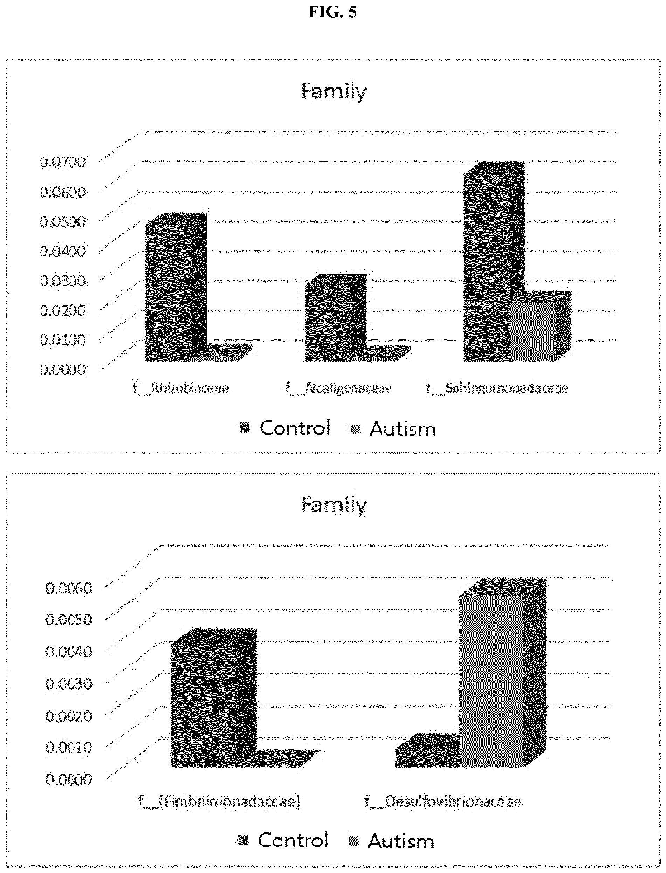

FIG. 5 is a result showing the distribution of bacteria-derived extracellular vesicles (EVs), which is significant in diagnostic performance at the family level by isolating bacteria-derived vesicles from urine of a patient with autism and a normal individual, and then performing a metagenomic analysis.

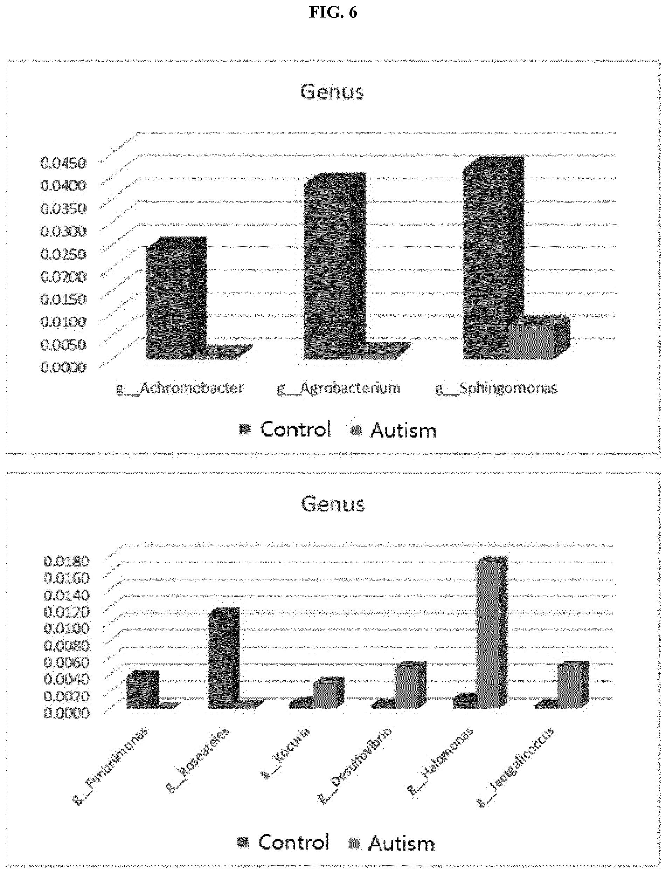

FIG. 6 is a result showing the distribution of bacteria-derived extracellular vesicles (EVs), which is significant in diagnostic performance at the genus level by isolating bacteria-derived vesicles from urine of a patient with autism and a normal individual, and then performing a metagenomic analysis.

### [Best Mode]

The present invention relates to a method of diagnosing autism through bacterial metagenomic analysis. The inventors of the present invention extracted genes from bacteria-derived extracellular vesicles using a normal individual and a subject-derived sample, performed metagenomic analysis thereon, and identified bacteria-derived extracellular vesicles capable of acting as a causative factor of autism.

The term "autism diagnosis" as used herein refers to determining whether a patient has a risk for autism, whether the autism has already occurred. The method of the present invention may be of use, clinically, to determine treatment by selecting the most appropriate treatment method through early diagnosis of autism.

The term "metagenome" as used herein refers to the total of genomes including all viruses, bacteria, fungi, and the like in isolated regions such as soil, the intestines of animals, and the like, and is mainly used as a concept of genomes that explains identification of many microorganisms at once using a sequencer to analyze non-cultured microorganisms. In particular, a metagenome does not refer to a genome of one species, but refers to a mixture of genomes, including genomes of all species of an environmental unit. This term originates from the view that, when defining one species in a process in which biology is advanced into omics, various species as well as existing one species functionally interact with each other to form a complete species. Technically, it is the subject of techniques that analyzes all DNAs and RNAs regardless of species using rapid sequencing to identify all species in one environment and verify interactions and metabolism. In the present invention, bacterial metagenomic analysis is performed using bacteria-derived extracellular vesicles isolated from urine.

More particularly, in the present invention, as a result of performing bacterial metagenomic analysis on extracellular vesicles present in subject-derived urine samples at a phylum level, the content of extracellular vesicles derived from bacteria belonging to the phylum *Armatimonadetes* and the phylum *Thermi* was significantly different between autism patients and normal individuals (see Example 4).

More particularly, in the present invention, as a result of performing bacterial metagenomic analysis on extracellular vesicles present in subject-derived urine samples at a class level, the content of extracellular vesicles derived from bacteria belonging to the class *Fimbriimonadia,* the class *Alphaproteobacteria,* and the class *Deinococci* was significantly different between autism patients and normal individuals (see Example 4).

More particularly, in the present invention, as a result of performing bacterial metagenomic analysis on extracellular vesicles present in subject-derived urine samples at an order level, the content of extracellular vesicles derived from bacteria belonging to the order *Fimbriimonadales,* the order *Rhizobiales,* the order *Sphingomonadales,* and the order *Desulfovibrionales* was significantly different between autism patients and normal individuals (see Example 4).

More particularly, in the present invention, as a result of performing bacterial metagenomic analysis on extracellular vesicles present in subject-derived urine samples at a family level, the content of extracellular vesicles derived from bacteria belonging to the family *Fimbriimonadaceae,* the family *Rhizobiaceae,* the family *Alcaligenaceae,* the family *Sphingomonadaceae,* and the family *Desulfovibrionaceae* was significantly different between autism patients and normal individuals (see Example 4).

More particularly, in the present invention, as a result of performing bacterial metagenomic analysis on extracellular vesicles present in subject-derived urine samples at a genus level, the content of extracellular vesicles derived from bacteria belonging to the genus *Fimbriimonas,* the genus *Achromobacter,* the genus *Roseateles,* the genus *Agrobacterium,* the genus *Sphingomonas,* the genus *Desulfovibrio,* the genus *Halomonas,* and the genus *Jeotgalicoccus* was significantly different between autism patients and normal individuals (see Example 4).

Through the results of the examples, it was confirmed that distribution variables of the identified bacteria-derived extracellular vesicles could be usefully used for the prediction of the onset of autism.

### [Mode of the Invention]

Hereinafter, the present invention will be described with reference to exemplary examples to aid in understanding of the present invention. However, these examples are provided only for illustrative purposes and are not intended to limit the scope of the present invention.

### [Examples]

### Example 1. Analysis of In Vivo Absorption, Distribution, and Excretion Patterns of Intestinal Bacteria and Bacteria-Derived Extracellular Vesicles

To evaluate whether intestinal bacteria and bacteria-derived extracellular vesicles are systematically absorbed through the gastrointestinal tract, an experiment was conducted using the following method. More particularly, 50 µg of each of intestinal bacteria and the bacteria-derived extracellular vesicles (EVs), labeled with fluorescence, were orally administered to the gastrointestinal tracts of mice, and fluorescence was measured at 0 h, and after 5 min, 3 h, 6 h, and 12 h. As a result of observing the entire images of mice, as illustrated in FIG. 1A, the bacteria were not systematically absorbed when administered, while the bacteria-derived EVs were systematically absorbed at 5 min after administration, and, at 3 h after administration, fluorescence was strongly observed in the bladder, from which it was confirmed that the EVs were excreted via the urinary system, and were present in the bodies up to 12 h after administration.

After intestinal bacteria and intestinal bacteria-derived extracellular vesicles were systematically absorbed, to evaluate a pattern of invasion of intestinal bacteria and the bacteria-derived EVs into various organs in the human body after being systematically absorbed, 50 µg of each of the bacteria and bacteria-derived EVs, labeled with fluorescence, were administered using the same method as that used above, and then, at 12 h after administration, blood, the heart, the lungs, the liver, the kidneys, the spleen, adipose tissue, and muscle were extracted from each mouse. As a result of observing fluorescence in the extracted tissues, as illustrated in FIG. 1B, it was confirmed that the intestinal bacteria were not absorbed into each organ, while the bacteria-derived EVs were distributed in the blood, heart, lungs, liver, kidneys, spleen, adipose tissue, and muscle.

### Example 2. Vesicle Isolation and DNA Extraction from Urine

To isolate extracellular vesicles and extract DNA, from urine, first, urine was added to a 10 ml tube and centrifuged at 3,500 x g and 4 °C for 10 min to precipitate a suspension, and only a supernatant was collected, which was then placed in a new 10 ml tube. The collected supernatant was filtered using a 0.22 µm filter to remove bacteria and impurities, and then placed in centripreigugal filters (50 kD) and centrifuged at 1500 x g and 4 °C for 15 min to discard materials with a smaller size than 50 kD, and then concentrated to 10 ml. Once again, bacteria and impurities were removed therefrom using a 0.22 µm filter, and then the resulting concentrate was subjected to ultra-high speed centrifugation at 150,000 x g and 4 °C for 3 hours by using a Type 90ti rotor to remove a supernatant, and the agglomerated pellet was dissolved with phosphate-buffered saline (PBS), thereby obtaining vesicles.

100 µl of the extracellular vesicles isolated from the urine according to the above-described method was boiled at 100 °C to allow the internal DNA to come out of the lipid and then cooled on ice. Next, the resulting vesicles were centrifuged at 10,000 x g and 4 °C for 30 minutes to remove the remaining suspension, only the supernatant was collected, and then the amount of DNA extracted was quantified using a NanoDrop sprectrophotometer. In addition, to verify whether bacteria-derived DNA was present in the extracted DNA, PCR was performed using 16s rDNA primers shown in Table 1 below.

**[Table 1]**

| Primer | | Sequence | SEQ ID NO. |
|---|---|---|---|
| 16S rDNA | 16S_V3_F | 5'-TCGTCGGCAGCGTC AGATGTGTATAAGAG ACAGCCTACGGGNGG CWGCAG-3' | 1 |
| | 16S_V4_R | 5'-GTCTCGTGGGCTCG GAGATGTGTATAAGA GACAGGACTACHVGG GTATCTAATCC-3' | 2 |

### Example 3. Metagenomic Analysis Using DNA Extracted from Urine

DNA was extracted using the same method as that used in Example 2, and then PCR was performed thereon using 16S rDNA primers shown in Table 1 to amplify DNA, followed by sequencing (Illumina MiSeq sequencer). The results were output as standard flowgram format (SFF) files, and the SFF files were converted into sequence files (.fasta) and nucleotide quality score files using GS FLX software (v2.9), and then credit rating for reads was identified, and portions with a window (20 bps) average base call accuracy of less than 99% (Phred score <20) were removed. After removing the low-quality portions, only reads having a length of 300 bps or more were used (Sickle version 1.33), and, for operational taxonomy unit (OTU) analysis, clustering was performed using UCLUST and USEARCH according to sequence similarity. In particular, clustering was performed based on sequence similarity values of 94% for genus, 90% for family, 85% for order, 80% for class, and 75% for phylum, and phylum, class, order, family, and genus levels of each OTU were classified, and bacteria with a sequence similarity of 97% or more were analyzed (QIIME) using 16S DNA sequence databases (108,453 sequences) of BLASTN and GreenGenes.

### Example 4. Autism Diagnostic Model Based on Metagenomic Analysis of Bacteria-Derived EVs Isolated from Urine

EVs were isolated from urine samples of 20 autism patients and 28 normal individuals, the two groups matched in age and gender, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

As a result of analyzing bacteria-derived EVs in urine at a phylum level, a diagnostic model developed using bacteria belonging to the phylum *Armatimonadetes* and the phylum *Thermi* as a biomarker exhibited significant diagnostic performance for autism (see Table 2 and FIG. 2).

**[Table 2]**

| | Control | | Autism | | t-test | | | | | |
|---|---|---|---|---|---|---|---|---|---|---|
| Taxon | Mean | SD | Mean | SD | p-valu e | Ratio | AUC | Accur acy | sensiti vity | spe cifi cit y |
| p_Armatimo nadetes | 0.003 8 | 0.006 5 | 0.000 0 | 0.000 1 | 0.004 6 | 0.00 | 0.74 | 0.73 | 0.57 | 0.9 5 |
| p_Thermi | 0.000 7 | 0.001 5 | 0.004 7 | 0.005 2 | 0.003 1 | 6.47 | 0.81 | 0.71 | 0.82 | 0.5 5 |

As a result of analyzing bacteria-derived EVs in urine at a class level, a diagnostic model developed using bacteria belonging to the class *Fimbriimonadia,* the class *Alphaproteobacteria,* and the class *Deinococci* as a biomarker exhibited significant diagnostic performance for autism (see Table 3 and FIG. 3).

**[Table 3]**

| | Control | | Autism | | t-test | | | | | |
|---|---|---|---|---|---|---|---|---|---|---|
| Taxon | Mean | SD | Mean | SD | p-valu e | Ratio | AUC | Accur acy | sensiti vity | sp eci fic ity |
| c_Fimbriimona dia | 0.003 8 | 0.006 5 | 0.000 0 | 0.000 1 | 0.004 6 | 0.00 | 0.74 | 0.73 | 0.57 | 0. 95 |
| c_Alphaproteo bacteria | 0.152 9 | 0.127 8 | 0.048 4 | 0.025 8 | 0.000 2 | 0.32 | 0.85 | 0.77 | 0.79 | 0. 75 |
| c_Deinococci | 0.000 7 | 0.001 5 | 0.004 7 | 0.005 2 | 0.003 1 | 6.47 | 0.81 | 0.71 | 0.82 | 0. 55 |

As a result of analyzing bacteria-derived EVs in urine at an order level, a diagnostic model developed using bacteria belonging to the order *Fimbriimonadales,* the order *Rhizobiales,* the order *Sphingomonadales,* and the order *Desulfovibrionales* as a biomarker exhibited significant diagnostic performance for autism (see Table 4 and FIG. 4).

**[Table 4]**

| | Control | | Autism | | t-test | | | | | |
|---|---|---|---|---|---|---|---|---|---|---|
| Taxon | Mean | SD | Mean | SD | p-valu e | Ratio | AUC | Accur acy | sensiti vity | spe cifi cit y |
| o_Fimbriimon adales | 0.003 8 | 0.006 5 | 0.000 0 | 0.000 1 | 0.004 6 | 0.00 | 0.74 | 0.73 | 0.57 | 0.9 5 |
| o_Rhizobiales | 0.056 9 | 0.053 9 | 0.010 5 | 0.009 2 | 0.000 I | 0.19 | 0.91 | 0.83 | 0.86 | 0.8 0 |
| o_Sphingomon adales | 0.062 9 | 0.064 2 | 0.019 8 | 0.015 9 | 0.001 8 | 0.31 | 0.81 | 0.73 | 0.82 | 0.6 0 |
| o_Desulfovibri onales | 0.000 6 | 0.001 4 | 0.005 3 | 0.005 9 | 0.001 9 | 9.66 | 0.82 | 0.81 | 0.93 | 0.6 5 |

As a result of analyzing bacteria-derived EVs in urine at a family level, a diagnostic model developed using bacteria belonging to the family *Fimbriimonadaceae,* the family *Rhizobiaceae,* the family *Alcaligenaceae*, the family *Sphingomonadaceae,* and the family *Desulfovibrionaceae* as a biomarker exhibited significant diagnostic performance for autism (see Table 5 and FIG. 5).

**[Table 5]**

| | Control | | Autism | | t-test | | | | | |
|---|---|---|---|---|---|---|---|---|---|---|
| Taxon | Mean | SD | Mean | SD | p-valu e | Ratio | AUC | Accur acy | sensiti vity | spe cifi cit y |
| f_Fimbriimon adaceae | 0.003 8 | 0.006 5 | 0.000 0 | 0.000 1 | 0.004 6 | 0.00 | 0.74 | 0.73 | 0.57 | 0.9 5 |
| f_Rhizobiacea e | 0.045 8 | 0.050 4 | 0.001 8 | 0.002 8 | 0.000 I | 0.04 | 0.96 | 0.88 | 0.89 | 0.8 5 |
| f_Alcaligenac cac | 0.025 3 | 0.040 9 | 0.001 2 | 0.002 9 | 0.004 4 | 0.05 | 0.79 | 0.75 | 0.82 | 0.6 5 |
| f_Sphingomon adaecae | 0.062 7 | 0.064 2 | 0.019 8 | 0.015 9 | 0.001 9 | 0.32 | 0.81 | 0.73 | 0.82 | 0.6 0 |
| f_Desulfovibri onaccae | 0.000 6 | 0.001 4 | 0.005 3 | 0.005 9 | 0.001 9 | 9.66 | 0.82 | 0.81 | 0.93 | 0.6 5 |

As a result of analyzing bacteria-derived EVs in urine at a genus level, a diagnostic model developed using bacteria belonging to the genus *Fimbriimonas,* the genus *Achromobacter,* the genus *Roseateles,* the genus *Agrobacterium,* the genus *Sphingomonas,* the genus *Kocuria*, the genus *Desulfovibrio*, the genus *Halomonas,* and the genus *Jeotgalicoccus* as a biomarker exhibited significant diagnostic performance for autism (see Table 6 and FIG. 6).

**[Table 6]**

| | Control | | Autism | | t-test | | | | | |
|---|---|---|---|---|---|---|---|---|---|---|
| Taxon | Mean | SD | Mean | SD | p-valu c | Ratio | AUC | Accur acy | scnsili vity | sp cci fic ity |
| g_Fimbrii monas | 0.003 8 | 0.006 5 | 0.000 0 | 0.000 1 | 0.004 6 | 0.00 | 0.74 | 0.73 | 0.57 | 0.9 5 |
| g_Achrom obacter | 0.024 2 | 0.039 6 | 0.000 5 | 0.001 5 | 0.003 8 | 0.02 | 0.83 | 0.77 | 0.86 | 0.6 5 |
| g_Roseatel es | 0.011 2 | 0.018 4 | 0.000 2 | 0.000 6 | 0.004 0 | 0.02 | 0.88 | 0.79 | 0.86 | 0.7 0 |
| g_Agrobac terium | 0.038 3 | 0.050 7 | 0.001 1 | 0.002 2 | 0.000 6 | 0.03 | 0.93 | 0.88 | 0.89 | 0.8 5 |
| g_Sphingo monas | 0.04 1 7 | 0.043 8 | 0.007 1 | 0.008 7 | 0.000 3 | 0.17 | 0.85 | 0.79 | 0.82 | 0.7 5 |
| g_Kocuria | 0.000 6 | 0.002 5 | 0.003 0 | 0.003 5 | 0.008 3 | 4.75 | 0.76 | 0.73 | 0.96 | 0.4 0 |
| g_Desulfov ibrio | 0.000 4 | 0.001 1 | 0.004 8 | 0.006 1 | 0.004 8 | 10.88 | 0.81 | 0.77 | 0.89 | 0.6 0 |
| g_Halomo nas | 0.001 2 | 0.003 1 | 0.017 2 | 0.022 8 | 0.005 4 | 14.61 | 0.91 | 0.79 | 0.93 | 0.6 0 |
| g_Jeotgalic occus | 0.000 3 | 0.000 9 | 0.005 0 | 0.005 1 | 0.000 7 | 14.70 | 0.84 | 0.81 | 0.93 | 0.6 5 |

### [Industrial Applicability]

The method of providing information for diagnosing autism through a bacterial metagenomic analysis according to the present invention may be used for diagnosing autism by performing a bacterial metagenomic analysis using normal individual-derived and subject-derived urine samples to analyze an increase or decrease in the content of specific bacteria-derived extracellular vesicles.

## Claims

1. A method of providing information for diagnosing autism, the method comprising:
(a) extracting DNAs from extracellular vesicles isolated from normal individual and subject urine samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR,
wherein process (c) comprises comparing an increase or decrease in content of extracellular vesicles derived from
one or more bacteria selected from the group consisting of the phylum *Armatimonadetes* and the phylum *Thermi;*
one or more bacteria selected from the group consisting of the class *Fimbriimonadia,* the class *Alphaproteobacteria,* and the class *Deinococci;*
one or more bacteria selected from the group consisting of the order *Fimbriimonadales*, the order *Rhizobiales,* the order *Sphingomonadales*, and the order *Desulfovibrionales;*
one or more bacteria selected from the group consisting of the family *Fimbriimonadaceae,* the family *Rhizobiaceae,* the family *Alcaligenaceae*, the family *Sphingomonadaceae,* and the family *Desulfovibrionaceae; or*
one or more bacteria selected from the group consisting of the genus *Fimbriimonas,* the genus *Achromobacter,* the genus *Roseateles,* the genus *Agrobacterium,* the genus *Sphingomonas,* the genus *Desulfovibrio*, the genus *Halomonas,* and the genus *Jeotgalicoccus,*
wherein in process (c), in comparison with the normal individual-derived sample, an increase in the content of the following is diagnosed as autism:
extracellular vesicles derived from bacteria of the phylum *Thermi,*
extracellular vesicles derived from bacteria of the class *Deinococci,*
extracellular vesicles derived from bacteria of the order *Desulfovibrionales,*
extracellular vesicles derived from bacteria of the family *Desulfovibrionaceae,* or
extracellular vesicles derived from one or more bacteria selected from the group consisting of the genus *Desulfovibrio*, the genus *Halomonas,* and the genus *Jeotgalicoccus;* or
wherein in process (c), in comparison with the normal individual-derived sample, a decrease in the content of the following is diagnosed as autism:
extracellular vesicles derived from bacteria of the phylum *Armatimonadetes,*
extracellular vesicles derived from one or more bacteria selected from the group consisting of the class *Fimbriimonadia* and the class *Alphaproteobacteria,*
extracellular vesicles derived from one or more bacteria selected from the group consisting of the order *Fimbriimonadales,* the order *Rhizobiales,* and the order *Sphingomonadales,*
extracellular vesicles derived from one or more bacteria selected from the group consisting of the family *Fimbriimonadaceae,* the family *Rhizobiaceae,* the family *Alcaligenaceae,* and the family *Sphingomonadaceae,* or
extracellular vesicles derived from one or more bacteria selected from the group consisting of the genus *Fimbriimonas,* the genus *Achromobacter*, the genus *Roseateles,* the genus *Agrobacterium,* and the genus *Sphingomonas.*

2. A method of diagnosing autism, the method comprising:
(a) extracting DNAs from extracellular vesicles isolated from normal individual and subject urine samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR,
wherein process (c) comprises comparing an increase or decrease in content of extracellular vesicles derived from
one or more bacteria selected from the group consisting of the phylum *Armatimonadetes* and the phylum *Thermi;*
one or more bacteria selected from the group consisting of the class *Fimbriimonadia*, the class *Alphaproteobacteria,* and the class *Deinococci;*
one or more bacteria selected from the group consisting of the order *Fimbriimonadales*, the order *Rhizobiales,* the order *Sphingomonadales,* and the order *Desulfovibrionales;*
one or more bacteria selected from the group consisting of the family *Fimbriimonadaceae,* the family *Rhizobiaceae,* the family *Alcaligenaceae*, the family *Sphingomonadaceae,* and the family *Desulfovibrionaceae; or*
one or more bacteria selected from the group consisting of the genus *Fimbriimonas,* the genus *Achromobacter,* the genus *Roseateles,* the genus *Agrobacterium,* the genus *Sphingomonas,* the genus *Desulfovibrio*, the genus *Halomonas,* and the genus *Jeotgalicoccus,*
wherein in process (c), in comparison with the normal individual-derived sample, an increase in the content of the following is diagnosed as autism:
extracellular vesicles derived from bacteria of the phylum *Thermi,*
extracellular vesicles derived from bacteria of the class *Deinococci,*
extracellular vesicles derived from bacteria of the order *Desulfovibrionales,*
extracellular vesicles derived from bacteria of the family *Desulfovibrionaceae,* or
extracellular vesicles derived from one or more bacteria selected from the group consisting of the genus *Desulfovibrio*, the genus *Halomonas,* and the genus *Jeotgalicoccus;* or
wherein in process (c), in comparison with the normal individual-derived sample, a decrease in the content of the following is diagnosed as autism:
extracellular vesicles derived from bacteria of the phylum *Armatimonadetes,*
extracellular vesicles derived from one or more bacteria selected from the group consisting of the class *Fimbriimonadia* and the class *Alphaproteobacteria,*
extracellular vesicles derived from one or more bacteria selected from the group consisting of the order *Fimbriimonadales,* the order *Rhizobiales,* and the order *Sphingomonadales,*
extracellular vesicles derived from one or more bacteria selected from the group consisting of the family *Fimbriimonadaceae,* the family *Rhizobiaceae,* the family *Alcaligenaceae,* and the family *Sphingomonadaceae,* or
extracellular vesicles derived from one or more bacteria selected from the group consisting of the genus *Fimbriimonas,* the genus *Achromobacter,* the genus *Roseateles,* the genus *Agrobacterium,* and the genus *Sphingomonas.*

## Patentansprüche

1. Verfahren zum Bereitstellen von Informationen zur Diagnose von Autismus, wobei das Verfahren Folgendes umfasst:
(a) Extrahieren von DNAs aus extrazellulären Vesikeln, die aus Harnproben eines unauffälligen Individuums und eines Patienten isoliert wurden;
(b) Durchführen einer Polymerase-Kettenreaktion (PCR) an der extrahierten DNA unter Verwendung eines Paars von Primern, die SEQ ID Nr: 1 und SEQ ID Nr: 2 aufweisen; und
(c) Vergleichen einer Erhöhung oder Verringerung des Gehalts von von Bakterien stammenden, extrazellulären Vesikeln der vom Patienten stammenden Probe mit der von einem unauffälligen Individuum stammenden Probe durch Sequenzierung eines Produkts der PCR,
wobei Prozess (c) das Vergleichen einer Erhöhung oder Verringerung des Gehalts von extrazellulären Vesikeln umfasst, die stammen von
einem oder mehreren Bakterien, die ausgewählt sind aus der Gruppe bestehend aus dem Phylum Armatimonadetes und dem Phylum Thermi;
einem oder mehreren Bakterien, ausgewählt aus der Gruppe bestehend aus der Klasse Fimbriimonadia, der Klasse Alphaproteobacteria und der Klasse Deinococci;
einem oder mehreren Bakterien, ausgewählt aus der Gruppe bestehend aus der Ordnung Fimbriimonadales, der Ordnung Rhizobiales, der Ordnung Sphingomonadales und der Ordnung Desulfovibrionales;
einem oder mehreren Bakterien, ausgewählt aus der Gruppe bestehend aus der Familie Fimbriimonadaceae, der Familie Rhizobiaceae, der Familie Alcaligenaceae, der Familie Sphingomonadaceae und der Familie Desulfovibrionaceae; oder
einem oder mehreren Bakterien, ausgewählt aus der Gruppe bestehend aus der Gattung Fimbriimonas, der Gattung Achromobacter, der Gattung Roseateles, der Gattung Agrobacterium, der Gattung Sphingomonas, der Gattung Desulfovibrio, der Gattung Halomonas und der Gattung Jeotgalicoccus,
wobei in Prozess (c), im Vergleich zur vom unauffälligen Individuum stammenden Probe, ein Anstieg des Gehalts des Folgenden als Autismus diagnostiziert wird:
extrazellulären Vesikeln, die von Bakterien des Phylums Thermi stammen,
extrazellulären Vesikeln, die von Bakterien der Klasse Deinococci stammen,
extrazellulären Vesikeln, die von Bakterien der Ordnung Desulfovibrionales stammen,
extrazellulären Vesikeln, die von Bakterien der Familie Desulfovibrionaceae stammen, oder
extrazellulären Vesikeln, die von einem oder mehreren Bakterien stammen, ausgewählt aus der Gruppe bestehend aus der Gattung Desulfovibrio, der Gattung Halomonas und der Gattung Jeotgalicoccus; oder
wobei in Prozess (c), im Vergleich zur vom unauffälligen Individuum stammenden Probe, eine Verringerung des Gehalts des Folgenden als Autismus diagnostiziert wird:
extrazellulären Vesikeln, die von Bakterien des Phylums Armatimonadetes stammen,
extrazellulären Vesikeln, die von einem oder mehreren Bakterien stammen, ausgewählt aus der Gruppe bestehend aus der Klasse Fimbriimonadia und der Klasse Alphaproteobacteria,
extrazellulären Vesikeln, die von einem oder mehreren Bakterien stammen, ausgewählt aus der Gruppe bestehend aus der Ordnung Fimbriimonadales, der Ordnung Rhizobiales und der Ordnung Sphingomonadales,
extrazellulären Vesikeln, die von einem oder mehreren Bakterien stammen, ausgewählt aus der Gruppe bestehend aus der Familie Fimbriimonadaceae, der Familie Rhizobiaceae, der Familie Alcaligenaceae und der Familie Sphingomonadaceae, oder
extrazellulären Vesikeln, die von einem oder mehreren Bakterien stammen, ausgewählt aus der Gruppe bestehend aus der Gattung Fimbriimonas, der Gattung Achromobacter, der Gattung Roseateles, der Gattung Agrobacterium und der Gattung Sphingomonas.

2. Verfahren zum Diagnostizieren von Autismus, wobei das Verfahren Folgendes umfasst:
(a) Extrahieren von DNAs aus extrazellulären Vesikeln, die aus Harnproben eines unauffälligen Individuums und eines Patienten isoliert wurden;
(b) Durchführen einer Polymerase-Kettenreaktion (PCR) an der extrahierten DNA unter Verwendung eines Paars von Primern, die SEQ ID Nr: 1 und SEQ ID Nr: 2 aufweisen; und
(c) Vergleichen einer Erhöhung oder Verringerung des Gehalts von von Bakterien stammenden, extrazellulären Vesikeln der vom Patienten stammenden Probe mit der von einem unauffälligen Individuum stammenden Probe durch Sequenzierung eines Produkts der PCR,
wobei Prozess (c) das Vergleichen einer Erhöhung oder Verringerung des Gehalts von extrazellulären Vesikeln umfasst, die stammen von
einem oder mehreren Bakterien, die ausgewählt sind aus der Gruppe bestehend aus dem Phylum Armatimonadetes und dem Phylum Thermi;
einem oder mehreren Bakterien, ausgewählt aus der Gruppe bestehend aus der Klasse Fimbriimonadia, der Klasse Alphaproteobacteria und der Klasse Deinococci;
einem oder mehreren Bakterien, ausgewählt aus der Gruppe bestehend aus der Ordnung Fimbriimonadales, der Ordnung Rhizobiales, der Ordnung Sphingomonadales und der Ordnung Desulfovibrionales;
einem oder mehreren Bakterien, ausgewählt aus der Gruppe bestehend aus der Familie Fimbriimonadaceae, der Familie Rhizobiaceae, der Familie Alcaligenaceae, der Familie Sphingomonadaceae und der Familie Desulfovibrionaceae; oder
einem oder mehreren Bakterien, ausgewählt aus der Gruppe bestehend aus der Gattung Fimbriimonas, der Gattung Achromobacter, der Gattung Roseateles, der Gattung Agrobacterium, der Gattung Sphingomonas, der Gattung Desulfovibrio, der Gattung Halomonas und der Gattung Jeotgalicoccus,
wobei in Prozess (c), im Vergleich zur vom unauffälligen Individuum stammenden Probe, ein Anstieg des Gehalts des Folgenden als Autismus diagnostiziert wird:
extrazellulären Vesikeln, die von Bakterien des Phylums Thermi stammen,
extrazellulären Vesikeln, die von Bakterien der Klasse Deinococci stammen,
extrazellulären Vesikeln, die von Bakterien der Ordnung Desulfovibrionales stammen,
extrazellulären Vesikeln, die von Bakterien der Familie Desulfovibrionaceae stammen, oder
extrazellulären Vesikeln, die von einem oder mehreren Bakterien stammen, ausgewählt aus der Gruppe bestehend aus der Gattung Desulfovibrio, der Gattung Halomonas und der Gattung Jeotgalicoccus; oder
wobei in Prozess (c), im Vergleich zur vom unauffälligen Individuum stammenden Probe, eine Verringerung des Gehalts des Folgenden als Autismus diagnostiziert wird:
extrazellulären Vesikeln, die von Bakterien des Phylums Armatimonadetes stammen,
extrazellulären Vesikeln, die von einem oder mehreren Bakterien stammen, ausgewählt aus der Gruppe bestehend aus der Klasse Fimbriimonadia und der Klasse Alphaproteobacteria,
extrazellulären Vesikeln, die von einem oder mehreren Bakterien stammen, ausgewählt aus der Gruppe bestehend aus der Ordnung Fimbriimonadales, der Ordnung Rhizobiales und der Ordnung Sphingomonadales,
extrazellulären Vesikeln, die von einem oder mehreren Bakterien stammen, ausgewählt aus der Gruppe bestehend aus der Familie Fimbriimonadaceae, der Familie Rhizobiaceae, der Familie Alcaligenaceae und der Familie Sphingomonadaceae, oder
extrazellulären Vesikeln, die von einem oder mehreren Bakterien stammen, ausgewählt aus der Gruppe bestehend aus der Gattung Fimbriimonas, der Gattung Achromobacter, der Gattung Roseateles, der Gattung Agrobacterium und der Gattung Sphingomonas.

## Revendications

1. Procédé de fourniture d'informations pour diagnostiquer l'autisme, le procédé comprenant :
(a) l'extraction d'ADN de vésicules extracellulaires isolées à partir d'échantillons d'urine provenant d'un individu normal et d'un sujet ;
(b) la réalisation d'une réaction en chaîne par polymérase (PCR) sur l'ADN extrait en utilisant une paire d'amorces présentant SEQ ID NO : 1 et SEQ ID NO : 2 ; et
(c) la comparaison d'une augmentation ou d'une diminution de la teneur en vésicules extracellulaires dérivées de bactéries de l'échantillon dérivé d'un sujet à celle d'un échantillon dérivé d'un individu normal par séquençage d'un produit de la PCR,
dans lequel un processus (c) comprend la comparaison d'une augmentation ou d'une diminution de teneur en vésicules extracellulaires dérivées de
une ou plusieurs bactéries sélectionnées dans le groupe consistant en le phylum Armatimonadetes et le phylum Thermi ;
une ou plusieurs bactéries sélectionnées dans le groupe consistant en la classe Fimbriimonadia, la classe Alphaproteobacteria et la classe Deinococci ;
une ou plusieurs bactéries sélectionnées dans le groupe consistant en l'ordre Fimbriimonadales, l'ordre Rhizobiales, l'ordre Sphingomonadales et l'ordre Desulfovibrionales ;
une ou plusieurs bactéries sélectionnées dans le groupe consistant en la famille Fimbriimonadaceae, la famille Rhizobiaceae, la famille Alcaligenaceae, la famille Sphingomonadaceae et la famille Desulfovibrionaceae ; ou
une ou plusieurs bactéries sélectionnées dans le groupe consistant en le genre Fimbriimonas, le genre Achromobacter, le genre Roseateles, le genre Agrobacterium, le genre Sphingomonas, le genre Desulfovibrio, le genre Halomonas et le genre Jeotgalicoccus,
dans lequel, dans le processus (c), par comparaison avec l'échantillon dérivé d'un individu normal, une augmentation de la teneur des éléments suivants est diagnostiquée comme autisme :
des vésicules extracellulaires dérivées de bactéries du phylum Thermi,
des vésicules extracellulaires dérivées de bactéries de la classe Deinococci,
des vésicules extracellulaires dérivées de bactéries de l'ordre Desulfovibrionales,
des vésicules extracellulaires dérivées de bactéries de la famille Desulfovibrionaceae, ou
des vésicules extracellulaires dérivées d'une ou plusieurs bactéries sélectionnées dans le groupe consistant en le genre Desulfovibrio, le genre Halomonas et le genre Jeotgalicoccus ; ou
dans lequel, dans le processus (c), par comparaison avec l'échantillon dérivé d'un individu normal, une diminution de la teneur des éléments suivants est diagnostiquée comme autisme :
des vésicules extracellulaires dérivées de bactéries du phylum Armatimonadetes,
des vésicules extracellulaires dérivées d'une ou plusieurs bactéries sélectionnées dans le groupe consistant en la classe Fimbriimonadia et la classe Alphaproteobacteria,
des vésicules extracellulaires dérivées d'une ou plusieurs bactéries sélectionnées dans le groupe consistant en l'ordre Fimbriimonadales, l'ordre Rhizobiales et l'ordre Sphingomonadales,
des vésicules extracellulaires dérivées d'une ou plusieurs bactéries sélectionnées dans le groupe consistant en la famille Fimbriimonadaceae, la famille Rhizobiaceae, la famille Alcaligenaceae, et la famille Sphingomonadaceae, ou
des vésicules extracellulaires dérivées d'une ou plusieurs bactéries sélectionnées dans le groupe consistant en le genre Fimbriimonas, le genre Achromobacter, le genre Roseateles, le genre Agrobacterium et le genre Sphingomonas.

2. Procédé de diagnostic de l'autisme, le procédé comprenant :
(a) l'extraction d'ADN de vésicules extracellulaires isolées à partir d'échantillons d'urine provenant d'un individu normal et d'un sujet ;
(b) la réalisation d'une réaction en chaîne par polymérase (PCR) sur l'ADN extrait en utilisant une paire d'amorces présentant SEQ ID NO : 1 et SEQ ID NO : 2 ; et
(c) la comparaison d'une augmentation ou d'une diminution de la teneur en vésicules extracellulaires dérivées de bactéries de l'échantillon dérivé d'un sujet à celle d'un échantillon dérivé d'un individu normal par séquençage d'un produit de la PCR,
dans lequel le processus (c) comprend la comparaison d'une augmentation ou d'une diminution de teneur en vésicules extracellulaires dérivées de
une ou plusieurs bactéries sélectionnées dans le groupe consistant en le phylum Armatimonadetes et le phylum Thermi ;
une ou plusieurs bactéries sélectionnées dans le groupe consistant en la classe Fimbriimonadia, la classe Alphaproteobacteria et la classe Deinococci ;
une ou plusieurs bactéries sélectionnées dans le groupe consistant en l'ordre Fimbriimonadales, l'ordre Rhizobiales, l'ordre Sphingomonadales et l'ordre Desulfovibrionales ;
une ou plusieurs bactéries sélectionnées dans le groupe consistant en la famille Fimbriimonadaceae, la famille Rhizobiaceae, la famille Alcaligenaceae, la famille Sphingomonadaceae et la famille Desulfovibrionaceae ; ou
une ou plusieurs bactéries sélectionnées dans le groupe consistant en le genre Fimbriimonas, le genre Achromobacter, le genre Roseateles, le genre Agrobacterium, le genre Sphingomonas, le genre Desulfovibrio, le genre Halomonas et le genre Jeotgalicoccus,
dans lequel, dans le processus (c), par comparaison avec l'échantillon dérivé d'un individu normal, une augmentation de la teneur des éléments suivants est diagnostiquée comme autisme :
des vésicules extracellulaires dérivées de bactéries du phylum Thermi,
des vésicules extracellulaires dérivées de bactéries de la classe Deinococci,
des vésicules extracellulaires dérivées de bactéries de l'ordre Desulfovibrionales,
des vésicules extracellulaires dérivées de bactéries de la famille Desulfovibrionaceae, ou
des vésicules extracellulaires dérivées d'une ou plusieurs bactéries sélectionnées dans le groupe consistant en le genre Desulfovibrio, le genre Halomonas et le genre Jeotgalicoccus ; ou
dans lequel, dans le processus (c), par comparaison avec l'échantillon dérivé d'un individu normal, une diminution de la teneur des éléments suivants est diagnostiquée comme autisme :
des vésicules extracellulaires dérivées de bactéries du phylum Armatimonadetes,
des vésicules extracellulaires dérivées d'une ou plusieurs bactéries sélectionnées dans le groupe consistant en la classe Fimbriimonadia et la classe Alphaproteobacteria,
des vésicules extracellulaires dérivées d'une ou plusieurs bactéries sélectionnées dans le groupe consistant en l'ordre Fimbriimonadales, l'ordre Rhizobiales et l'ordre Sphingomonadales,
des vésicules extracellulaires dérivées d'une ou plusieurs bactéries sélectionnées dans le groupe consistant en la famille Fimbriimonadaceae, la famille Rhizobiaceae, la famille Alcaligenaceae, et la famille Sphingomonadaceae, ou
des vésicules extracellulaires dérivées d'une ou plusieurs bactéries sélectionnées dans le groupe consistant en le genre Fimbriimonas, le genre Achromobacter, le genre Roseateles, le genre Agrobacterium et le genre Sphingomonas.
